Europäisches Patentamt

European Patent Office

Office européen des brevets

(19)

(11) Numéro de publication: **0 201 651**
**B1**

(12) **FASCICULE DE BREVET EUROPEEN**

(45) Date de publication du fascicule du brevet: **04.04.90**

(51) Int. Cl.⁵: **A 61 F 2/42,** A 61 F 2/46

(21) Numéro de dépôt: **85400958.6**

(22) Date de dépôt: **15.05.85**

(54) **Implant prothétique pour une articulation métatarso-phalangienne et dispositif de fraisage utilisé pour sa mise en place.**

(43) Date de publication de la demande:
**20.11.86 Bulletin 86/47**

(45) Mention de la délivrance du brevet:
**04.04.90 Bulletin 90/14**

(84) Etats contractants désignés:
**AT BE CH DE GB IT LI LU NL SE**

(56) Documents cités:
**WO-A-79/00739**
**DE-A-2 852 738**
**FR-A-2 094 904**
**US-A-4 156 296**
**US-A-4 231 121**
**US-A-4 242 759**

**MEDIZINISCH-ORTHOPÄDISCHE TECHNIK, vol. 102, no. 4, juillet-aou7t 1982, pages 103-108, Stuttgart; F. SUTTER et al.: "Entwicklung einer Hohlzylinder-Schalenprothese als Femurkopfersatz"**

(73) Titulaire: **Lelievre, Jean-François**
**11 rue des Blagis**
**F-92340 Bourg La Reine (FR)**
(73) Titulaire: **MEDICALEX Société Anonyme dite:**
**39, Rue Croulebarbe**
**F-75013 Paris (FR)**

(72) Inventeur: **Lelievre, Jean-François**
**11 rue des Blagis**
**F-92340 Bourg La Reine (FR)**
Inventeur: **Levy, Aldo**
**10 rue Vandrezanne**
**F-75013 Paris (FR)**

(74) Mandataire: **Cournarie, Michèle et al**
**OFFICE BLETRY R.& G. ROGER-PETIT & R.BLETRY 2, boulevard de Strasbourg**
**F-75010 Paris (FR)**

EP 0 201 651 B1

Courier Press, Leamington Spa, England.

## Description

Depuis une vingtaine d'années on se pose le problème de la reconstitution prothétique ou non des articulations métatarsophalangiennes qu'ill s'agisse d'arthrose ou de résection chirurgicale malencontreuse de la tête de premier métatarsien.

La première idée a été l'auto-greffe vissée mais le greffon s'est lysé dans 100% des cas.

Depuis 1969, divers implants prothétiques ont été envisagés.

1 — *Implant de silastic*

soit uniquement phalangien, mais on note une lyse de la diaphyse ou une usure de la tête métatarsienne faisant vis-à-vis,

soit implant de type charnière, mais il s'use, se brise et l'os avoisinant se lyse, laissant un déficit osseux considérable au bout de 3 à 4 ans — entre 20 et 30% du volume osseux local.

2 —*Prothése "Téflon"-metal* cimentée, en deux parties, "Téflon" étant une marque déposée pour des résines fluoro-carbonées. On observe aussi une lyse osseuse au contact des éléments prothétiques. Les lyses osseuses sont toujours problématiques à réparer.

3 — Ainsi, qu'ill s'agisse d'auto-greffe vissée ou d'implant prothétique, on assiste à une lyse osseuse. Il est logique de penser que ce phénomène est directement lié à l'importance des contraintes qui s'exercent au niveau de la première articulation métatarso-phalangienne, d'où l'idée de la *prothèse du Docteur REGNAULD* en "Téflon". Cette prothèse n'est qu'une mince cupule sans prise osseuse. Etant fort mince : 1 mm, elle ne donne pas prise aux contraintes; s'il faut la retirer, son faible encombrement fait qu'il n'y a pas de déficit régional.

Toutefois l'absence de fixation osseuse a pour conséquence qu'elle migre souvent partiellement hors de la zone d'efficacité.

Le document US—A—4 231 121 décrit des prothèses métacarpiennes et phalangiennes qui sont maintenues en place par enfoncement d'une tige dans le canal médullaire de l'os correspon-dant, ce qui entraîne progressivement une lyse osseuse importante. Dans ce document, la prothèse phalangienne prisuite une surface externe concave et la métacarpienne une surface externe convere de même couchere que celle de la surface concave.

Pour pallier ces inconvénients (lyse ou migration), la présente invention a pour objet un implant prothétique de petite taille, donc ne recevant que de faibles contraintes, mais suffisamment fixé à l'os pour ne point migrer.

Cet implant comprend une prothère phalangienne présentant une surface externe concave et une prothèse métatarsienne présentant une surface externe convexe de même courbure que celle de la surface concave, caractérisé en ce que la prothèse phalangienne (1) est sous forme d'une cupule comprenant une partie cylindrique (2) creuse liée à un fond (3) dont la face externe (4) est concave pour remplir la fonction du cartilage articulaire disparu, cette cupule étant destinée à coiffer la base de la première phalange (8) de l'orteil à traiter par enfoncement à frottement dur de sa partie cylindrique dans des logements de dimensions correspondantes formés au moyen d'une tréphine dans la corticale, voire les ostéophytes (9) entourant ladite base de la première phalange, la cupule (1) étant ainsi fixée dans l'os par une portion, de sa partie cylindrique (2); et en ce que la prothèse métatarsienne (5) comprend une partie en forme de calotte sphérique (6) destinée à faire office de cartilage de articulaire et surmontant une partie en forme de cylindre tronqué (7) à laquelle elle est liée, cette prothèse métatarsienne étant destinée à être fixée sur la face antérodorsale de la tête (10) du métatarsien (11) correspondant audit orteil par ledit cylindre tronqué (7), qui est enfoncé à cet effet à frottement dur et est ainsi bloqué dans un logement de dimensions correspondantes, formé au moyen d'une tréphine dans la zone dorsale de la tête dudit métatarsien, métatarsienne comporte pour une meilleure fixation, un axe à contour strié disposé centralement sensiblement prependicu-lairement à la base de la prothèse.

Les prothèses peuvent être réaliséesen une résine fluorocarbonée telle que celle de la marque déposée "Téflon", qui est bien supportée par l'organisme, ou en fibres de carbone (qui s'intègrent à l'os), ou même en un métal toléré par l'organisme tel que l'acier inoxydable ou un alliage cobalt-molybdène-chrome, par exemple de la marque déposée "Vitallium". la prothèse métatarsienne est de préférence métallique.

Le présent implant comprend donc deux prothèses, qui ne sont pas scellées, mais, sont simplement coincées dans l'os par frottement dur. En outre, elles ne sont ni lourdes ni volumineuses; l'épaisseur de leus parois est de l'ordre du millimètre, sauf celle du fond plan-concave de ladite cupule, qui varie de 0,1 à 1 milllimètre au centre, à 2 à 3 millimètres sur le pourtour.

La partie cylindrique de la cupule encapuchonne la métaphyse ou la partie distale de la diaphyse de la première phalange, selon que l'on a décidé ou non d'un raccourcissement opératoire de la première phalange. La cupule est enfoncée suivant l'axe de la première phalange jusqu'à ce que son fond bute sur cette phalange.

La prothèse métatarsienne est indispensable lorsque le cartilage articulaire de la tête du métatarsien est très abîmé. Dans ce cas, il est impor-tant de ne réséquer que très peu de cette tête et seulement dans sa zone antéro-dorsale; il faut en effet éviter les contraintes qui feraient fondre l'os sur la face plantaire de ladite tête, qui vient appuyer sur le sol au cours de la marche. En conséquence, l'axe d'enfoncement de la prothèse métatarsienne est dévié vers le haut par rapport à l'axe du métatarsien, par exemple de 45°. Cet angle est favorable, car les mouvements fondamentaux de l'articulation métatarso-phalangienne se font de part et d'autre de cet axe à 45°. Avec cette position de la prothèse métatarsienne, on maintient de façon optimale les deux fonctions de la tête métatarsienne: la trame osseuse sur sa face inférieure continue à assurer le soutien du poids du corps, tandis que la prothèse sur sa face supérieure permet la motilité habituelle de l'articulation.

L'invention a également pour objet un dispositif de fraisage pour le positionnement d'un implant prothétique tel que la cupule d'interposition susindiquée.

Jusqu'à présent, on utilisait en chirurgie osseuse des implants notamment en forme de calotte sphérique. De tels implants, également dénommés cupules d'interposition, sont munis sur leur pourtour de trous qui permettent leur fixation sur une partie de l'os à réparer, laquelle a été préalablement taillée de façon appropriée. Ces cupules d'interposition en forme de calotte sphéreique présentent le désavantage de déraper et ne permenttent donc pas de réaliser une ostéosynthèse fiable.

On a maintenant trouvé un nouveau procédé de positionnement d'un implant prothétique. Ce procédé consiste à ménager sur l'extrémité de l'un des deux os de la région articulaire à réparer une saillie de forme cylindrique à l'aide d'un dispositif de fraisage, à déposer sur ladite saillie cylindrique de l'os une cupule présentant un logement cylindrique correspondant et une face courbe destinée à venir en contact avec une prothèse sin l'extrémité en regard de l'autre os do ladite région articulaire, et à remettre en place cet autre os.

Ce procédé est particulièrement approprié pour réparer les articulations de type énarthrose et notamment les articulations des pieds.

Le dispositif de fraisage utilisé comprend une tige (25) munie à l'une de ses extrémités d'une poignée (26) et comportant à son autre extrémité und fraise tubulaire (30) constituée d'un tube présentant sur l'un de ses bords une série de dents (31) destinées au taillage, caractérisé en ce qu'il comprend en outre une fraise centrale interne (27) emmanchée sur le tige (25) et constituée d'un cylindre plein (28) placé à l'intérieur de la fraise tubulaire (30), lié à cette fraise et comportant sur sa face extrême une série de dents (29) s'étendant radialement; et éventuellement un foret central (32) monté dans la fraise centrale interne (27) et fixé dans la tige (25).

Des formes particulières d'exécution de l'implant prothétique suivant l'invention et du dispositif de fraisage utilisé pour son positionnement vont être décrites ci-après, à titre d'exemples purement indicatifs et nullement limitatifs, en référence au dessin annexé sur lequel.

Les figures 1a, 1b et 1c sont, respectivement, une vue en plan, une vue en élévation et une vue en coupe verticale axiale de la prothèse phalangienne d'un implant prothétique de l'articulation métatarso-phalangienne du gros orteil.

Les figures 2a, 2b et 2c sont, respectivement, une vue en plan, une vue en élévation et une vue en coupe verticale axiale de la prothèse métatarsienne de cet implant.

La figure 3 est une vue schématique en élévation montrant l'implant prothétique mis en place dans le région de l'articulation métatarso-phalangienne de gros orteil, abstraction étant faite des tissus du pied.

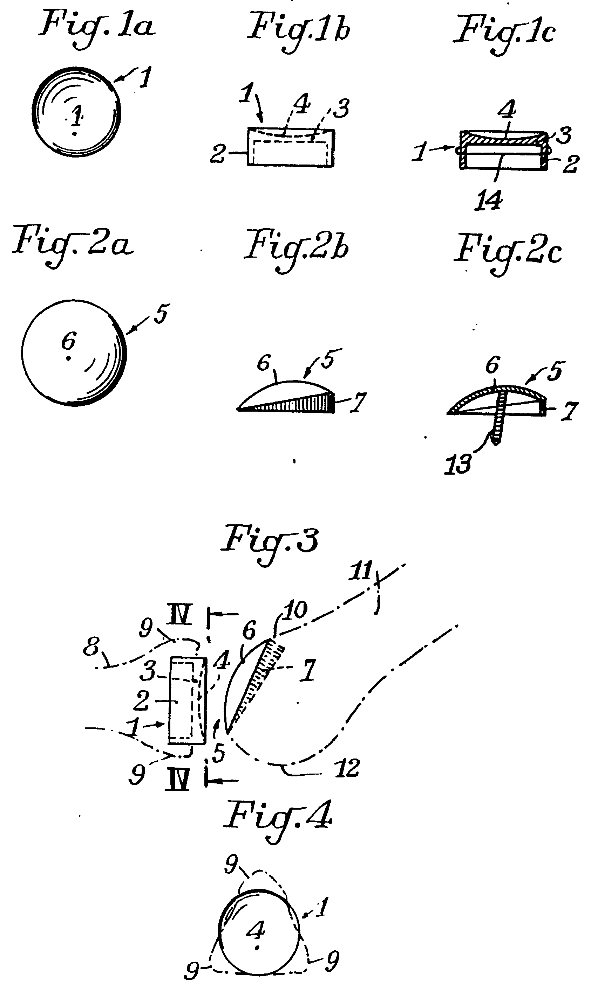

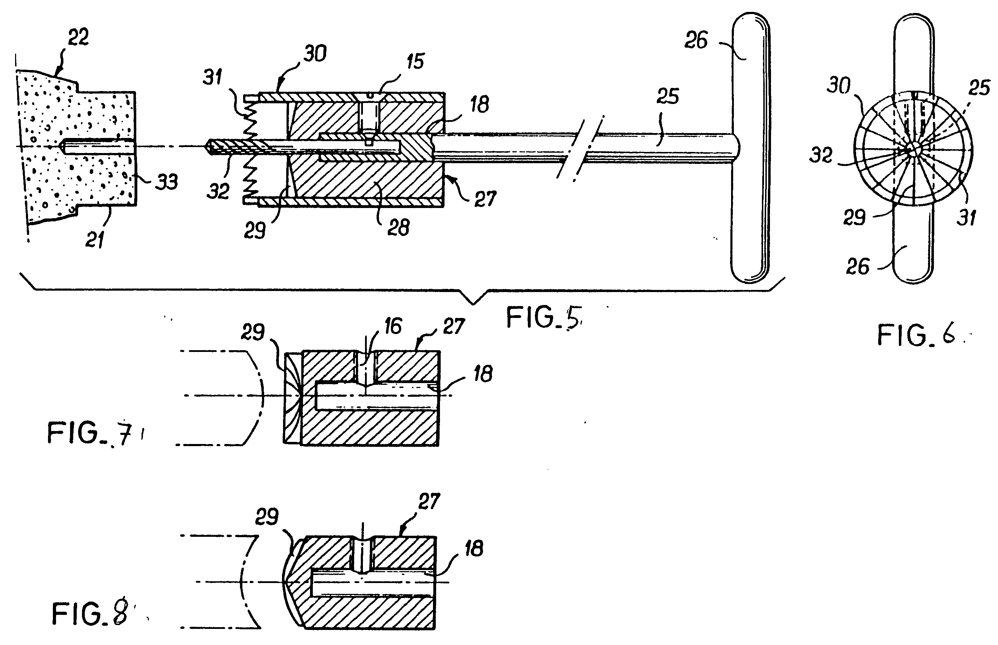

Le figure 4 est une vue en coupe suivant le ligne IV—IV de la figure 3.

La figure 5 est une vue en élévation et en coupe du dispositif de fraisage selon l'invention et de l'os après fraisage.

La figure 6 est une vue en bout du dispositif de fraisage selon la figure 5.

Les figures 7 et 8 sont des variantes de réalisation de la fraise centrale interne.

La prothèse phalangienne (figures 1a, 1b et 1c) est une cupule 1, par exemple moulée en "Téflon", qui comprend une partie cylindrique 2 liée à un fond 3, dont la face externe 4 est concave. La partie cylindrique 2 est creuse.

La prothèse métatarsienne 5 (figures 2a, 2b et 2c) comprend une partie en forme de calotte sphérique 6 surmontant une partie en forme de cylindre tronqué 7 à laquelle elle est liée. La surface interne de ce cylindre est striée. La prothèse métatarsienne peut aussi être réalisée par moulage en "Téflon", mais elle est de préférence métallique.

La prothése phalangienne I a un diamètre correspondant approximativement à celui de la base de la première phalange 8 du gros orteil, abstraction faite des ostéophytes, entourant cette base. Pour mettre en place cette prothèse, on utilise une tréphine, dont les diamètres interne et externe correspondent à ceux de la cupule 1. Avec cette tréphine, on creuse, parallèlement à la direction de l'axe de la premiére phalange 8, des logements dans la corticale et dans les ostéophytes 9 présents autour de la base de la phalange, logements dont la longueur doit être égale à celle de la partie cylindrique 2 de la cupule. On enfonce ensuite celle-ci à frottement dur dans lesdits logements par sa partie cylindrique 2, dont l'épaisseur est égale à celle de ces logements, jusq'à ce que la face interne du fond 3 de la cupule vienne au contact de la face en bout de la première phalange 8 (figure 3). On voit à la figure 4 que la cupule 1, qui coiffe la base de la première phalange 8, n'est enfonce et fixée dans l'os que dans la région des ostéophytes 9, donc par une fraction seulement de sa partie cylindrique 2.

La prothèse métatarsienne 5 est mise en place sur la face antéro-dorsale de la tête 10 du premier métatarsien 11. Elle est enfoncée dans cette tête par se partie en forme de cylindre tronqué 17 suivant un axe, qui dévié vers le haut, par exemple de 30 à 45°, par rapport à l'axe du premier métatarsien. Comme dans la cas de la prothèse phalangienne, un logement permettant unfoncement à frottement dur est préparé au moyen d'un tréphine ayant des diamètres interne et externe correspondant à ceux de la partie en forme de cylindre tronqué 7. L'enfoncement est effectué jusqu'à ce que la partie en forme de calotte sphérique 6 soit an contact de la tête 10 du premier métatarsien 11. La face plantaire 12 de la tête reste libre.

Les courbures de fond concave 4 de la cupule 1 et de la calotte sphérique 6 de la prothèse métatarsienne sont identiques pour permettre un pivotement relatif convenable.

Sur la figure 3, les os sont représentés schématiquement en traits mixtes et la phalange 8 est nettement séparée du premier métatarsien 11 pour montrer les prothèse avec plus de clarté.

La prothèse métatarsienne 5 comporte de préférence, pour consolider sa fixation, un axe à contour strié 13 représenté à la figure 2c et disposé contralement sensiblement prependicu-lairement à la base en forme de cylindre tronqué 7 de la prothèse. Une boucle de blocage 14 (figure 1c) en fil d'acier peut renforcer la fixation de la prothèse phalangienne, qu'elle traverse diamétralement et dont les extrémités sont nouées sur la surface latérale de la prothèse.

Comme montré à la figure 5, le procédé peut consister à réaliser une saillie cylindrique 21 sur une extrémité de l'os à réparer 22, à disposer sur ladite saillie 21 une cupule 1 présentant un logement cylindrue 2 correspondant à la saillie et une face courbe externe 4 concave destinée à venir en contact avec une prothèse metatarsienne 5 posée l'extrémité de l'autre os.

La saillie cylindrique osseuse 21 est réalisée à l'aide du dispositif de fraisage représenté sur les figures 5 et 6.

Ce dispositif de fraisage est constitué d'une tige 25 munie à l'une de ses extrémités d'une poignée 26 et à l'autre extrémité de moyens de fraisage proprement dits; ces moyens de fraisage comprennent:

1) une fraise contrale interne 27 constituée d'un cylindre plein 28 présentant sur sa face extrême une série de dents 29 s'étendant radialement sur ladite face extrême. Ledit cylindre est évidé dans sa partie axiale, l'évidement 18 étant tel qu'il permet l'emmanchement du cylindre sur la tige 15;

2) une fraise tubulaire 30 constituée d'un tube fixé sur la fraise centrale 27, par example au moyen d'une vis 15 engagée dans un trou taraudé 16 du cylindre 28 de la fraise centrale interne; le tube 30 présente sur l'un de ses bords une série de dents 31 destinées ua taillage.

La fraise centrale 27 peut comporter également un trou axial dans lequel est fixé on foret central 32.

Les dents 31 de la fraise tubulaire 30 et celles 29 de la fraise centrale interne sont dirigées du même côté.

Les dents des deux fraise sont soit en dents de scie, soit à pans coupés.

Par rotation du dispositif de fraisage, centré à l'aide du foret 32 sur la partie à traiter 22, on réalise une saillie plus ou moins haute selon la distance qui sépare les dents de la fraise tubulaire 30 de celles de la fraise centrale interne 27; la fraise interne 27 peut en effet être positionnée plus ou moins profondément dans le fraise tubulaire 30. Lorsque les dents sont à pans coupés, la rotation doit être alternative.

Grâce à la fraise centrale interne 27, la partie supérieure 33 de la saille osseuse 21 est convenablement nivelée en une surface plane, convexe ou concave, selon que les dents de la fraise centrale interne 27 sont dans un plan ou sont agencées de manière à définir par rotation une surface convexe ou concave, comme représentée sur les figures 7 et 8, les traits en pointillés sur ces figures représentant la forme des sailles correspondantes obtenues avec des fraises internes concaves ou convexes.

L'extrémité de la paroi de corps cylindrique peut être coupée selon un plan paralléle à face interne du fond dudit corps ou selon un plan oblique.

La cupule peut être en une matière quelconque couramment utilisée en chirurgie osseuse, telle que exemple métal, plastique, carbone, os, céramique médicale etc.

Le dispositif de fraisage selon l'invention est en acier inoxydable ou en tout autre métal ou alliage, par exemple titane, chrome-cobalt, carbone, etc.

Des modifications de détail du domaine des équivalents techniques peuvent être apportées à l'implant prothétique et au dispositif de fraisage décrits ci-dessus, sans que l'on sorte pour cela du domaine de l'invention.

## Revendications

1. Implant prothétique pour une articulation métatarsophalangienne, comprenant une prothèse phalangienne présentant une surface externe concave et une prothèse metatarsienne présentant une surface externe convexe de méme couchere que ladite surface concave, caractérisé en ce qur la prothèse phalangienne (1) est sous forme d'une cupule comprenant une partie cylindrique (2) creuse liée à un fond (3) dont la face externe (4) est concave pour remplir la fonction du cartilage articulaire disparu, cette cupule étant destinée à coiffer la base de la première phalange (8) de l'orteil à traiter par enfoncement à frottement dur de sa partie cylindrique dans des logements de dimensions correspondantes formés au moyen d'une tréphine dans la corticale, voire les ostéophytes (9) entourant ladite base de la pre-miére phalange, la cupule (1) ainsi fixée dans l'os par une portion de sa partie cylindrique (2); et en ce que la prothèse métatarsienne (5) comprend une partie en forme de calotte sphérique (6) destinée à faire office de cartilage articulaire et surmontant une partie en forme de cylindre tronqué (7) à laquelle elle est liée, cette prothèse métatarsienne étant destinée à être fixée sur la face antéro-dorsale de la tête (10) du métatarsien (11) correspondant audit orteil par ledit cylindre tronqué (7), qui est enfoncé à cet effet à frottement dur et est ainsi bloqué dans un logement de dimensions correspondantes, formé au moyen d'une tréphine dans la zone dorsale de la tête dudit métatarsien.

2. Implant prothétique suivant la revendication 1, caractérisé en ce que ladite partie en forme de cylindre tronqué (7) de la prothèse métatarsienne (5) est striée intérieurement.

3. Implant prothétique suivant la revendication 1 ou 2, caractérisé en ce que la prothése phalangienne (1) et la prothèse métatarsienne (5) sont réalisées en résines fluorocarbonées, en fibres de carbone ou en un métal toléré par l'organisme.

4. Implant prothétique suivant la revendication 3, caractérisé en ce que la prothèse métatarsienne est métallique.

5. Implant prothétique suivant l'une quelconque des revendications 1 à 4, caractérisé en ce qu'au moins une boucle de blocage, en fil d'acier, est associée à la prothèse phalangienne.

6. Implant prothétique suivant l'une quelconque des revendications 1 à 5, caractérisé en ce que la prothèse métatarsienne comporte un axe de fixation à contour strié disposé centralement sensiblement perpendiculairement à la base de la prothèse.

7. Dispositif de fraisage pour chirurgie osseusse et notamment pour la mise en place de l'implant prothétique suivant la revendication 1, comprenant une tige (25) munie à l'une de ses extrémités d'une poignée (26) et comportant à son autre extrémité une fraise tubulaire (30) constituée d'un tube présentant sur l'an de ses bords une série de dents (31) destinées au taillage, caractérisé en ce qu'il comprend en outre une fraise centrale interne (27) emmanchée sur la tige (25) et constituée d'un cylindre plein (28) placé à intérieur de la fraise tubulaire (30), lié à cette fraise et comportant sur sa face extrême une série de dents (29) s'étendant radialement; et éventuellement un foret central (32) monté dans la fraise centrale interne (27) et fixe dans la tige (25).

8. Dispositif selon la revendication 7, caractérisé en ce que les dents de la fraise tubulaire (30) et celles de la fraise centrale interne (27) sont dirigées du même côte.

9. Dispositif selon l'une quelconque des revendications 7 ou 8, caractérisé en ce que les dents des deux fraises sont en dents de scie ou à pans coupés.

10. Dispositif selon l'une quelconque des revendications 7 à 9, caractérisé en ce que la fraise tubulaire (30) est montée sur la fraise centrale interne (27) au moyen d'une vis (15) engagée dans un trou taraudé (16) du cylindre de la fraise centrale interne, ladite fraise interne (27) étant positionnée plus ou moins profondément dans la fraise tubulaire (30).

**Patentansprüche**

1. Prothetisches Implantat für ein metatarsales phalangiales Gelenk (Mettelfuβ/Zehen-Gelenk) mit einer phalangialen Prothese mit konkaver Außenfläche und einer metatarsalen Prothese mit konvexer Außenfläche gleicher Krümmung wie dei genannte konkave Fläche, dadurch gekenzeichnet, daβ die phalangiale Prothese (1) die Form eines Näpfchens mit einem hohlzylindrischen Teil (2) hat, das mit einem Boden (3) versehen ist, dessen Außfläche (4) konkav ist um die Funktion des fehlenden Gelencknorpels zu übernehmen, wobie dieses Näpfchen dazu bestimmt ist, die Basis des ersten Zehengliedes (8) der su behandelnden Zehe durch Einpressen seines zylindrischen Teils in Ausnehmungen entsprechender Abmessungen unter starker Reibung zu umschließen, welche Aushehmungen mittels einer Hohlfräse in der Knochenhaut und selbst in den die genannte Basis der ersten Zehengliedes umgebenden Osteophyten (9) erzeugt sind, so daβ das Näpfchen (1) auf diese Weise durch einen Abschnitt eines zylinderischen Teils (2) in dem Knochen fixiert ist; und daβ die metatarsale Prothese (5) ein Teil in Form einer Kugelkalotte (6) unfaβt, das dazu dient, den Gelekknorpel zu ersetzen und ein Teil (7) in Form eines Zylinderstumpfes, mit dem es verdunden ist, überragt, wobie diese metatarsale Prothese vorgesehen ist, über den Zylinderstumpf (7) und der anterodorsalen Fläche des der genannten Zehe entsprechenden Kopfes (10) des Metatarsus (11) defestigt zu werden, welcher Zylinderstumpf hierzu unter starker Reibung eingepreβt wird und somit in einer Ausnehmung entsprechender Abmessungen festgelegt ist, welche Ausnehmung mittels einer Hohlfräse im dorselan Bereich des metatarsalen Kopfes erzeugt wird.

2. Prothetisches Implantat nach Anspruch 1, dadurch gekennzeichnet, daβ das genannte zylinderstumpfförmige Teil (7) der metatarsalen Prothese (5) innenseitig gerieft ist.

3. Prothetisches Implantat nach Anspruch 1 oder 2, dadurch gekennzeichnet daβ die phalangiale Prothese (1) und die metatarsale Prothese (5) aus Fluorkohlenstoffharzen, aus Kohlefasern oder aus einem körperverträglichen Metall hergestellt sind.

4. Prothetisches Implantat nach Anspruch 3, dadurch gekennzeichnet, daβ die metatarsale Prothese aus Metall ist.

5. Prothetisches Implantat nach einem der Ansprüche 1 bis 4, dadurch gekennzeichnet, daβ der phalangialen Prothese mindestens eine Sperrschleife aus Stahldraht zugeordent ist.

6. Prothetisches Implantat nach einem der Ansprüche 1 bis 5, dadurch gekennzeichnet, daβ die metatarsale Prothese eine Befestigungsachse mit geriefter Mantelfläche umfaβt, die zentral zumindest annähernd rechtwinkelig zu der Basis der Prothese angeordnet ist.

7. Fräsvorrichtung für die Knockenchirurgie und insbesondere für das Einsetzen eines prothetischen Implantats nach Anspruch 1, mit einem Schaft (25), der an einem seiner Enden mit einem Handgriff (26) versehen ist und an seinem anderen Ende einen rohrförmigen Fräser (30) aufweist, der aus einem Rohr besteht, das an einem seiner Ränder eine Serie von Zähnen (31) zum Schneiden hat, dadurch gekennzeichnet, daβ sie außerdem einen zentralen inneren Fräser (27) umfaβt, der auf den Schaft (26) aufgepreβt ist und aus einem im Inneren des rohrförmigen Fräsers (30) angeordneten und mit diesem verbundenen Vollzylinder (28) besteht, der an seiner Stirnfläche eine Anzahl von sich radial erstreckenden Zahnen (29) hat; und ggf, einen Zentrumsbohrer (32), der in dem zentralen inneren Fräser (27) montiert und in dem Schaft (25) befestigt ist.

8. Vorrichtung nach Anspruch 7, dadurch gekennzeichnet, daß die Zähne des rohrförmigen Fräsers (30) und diejenigen des zentralen inneren Fräsers (27) nach derselben Seite gerichtet sind.

9. Vorrichtung nach einem der Ansprüche 7 oder 8, dadurch gekennzeichnet, daß die Zähne der zwei Fräser sägezahnfömig oder mit schrägen Kanten gestaltet sind.

10. Vorrichtung nach einem der Ansprüche 7 bis 9, dadurch gekennzeichnet, daß der rohrförmige Fräser (30) auf dem zentralen inneren Fräser (27) mittels einer Schraube (15), die in ein Gewindeloch (16) in dem Zylinder des zentralen inneren Fräsers eingreift, befestigt ist, wobie der genannte innere Fräser (27) mehr oder weniger tief in dem rohrförmigen Fräser (30) positioniert ist.

## Claims

1. Prosthetic implant for a phalangeal prosthesis having a concave outer surface and a metatarsal prosthesis having a convex outer surface of the same curvature as that of the concave surface, characterized in that the phalangeal prosthesis (1) is in the form of a cupula having a hollow cylindrical part (2) connected to a base (3), whose outer face (4) is concave in order to fulfil the function of the articular cartilage which has disappeared, said cupula serving to cover the bottom of the first phalanx (8) of the toe to be treated by hard frictional driving in of its cylindrical part into the recesses with corresponding dimensions formed by means of a trephine in the corticalis, namely the osteophytes (9) surrounding said bottom of the first phalanx, the cupula (1) thus being fixed in the bone by a portion of its cylindrical part (2) and in that the metatarsal prosthesis (5) comprises a part shaped like a spherical cap (6) which will serve as the articular cartilage and which surmounts a part shaped like a truncated cylinder (7) to which it is connected, said metatarsal prosthesis serving to be fixed to the anterodorsal face of the head (10) of the metatarsal bone (11) corresponding to said toe by said truncated cylinder (7), which for this purpose is driven in with hard friction and is thus locked in a recess having corresponding dimensions and formed by means of a trephine in the dorsal region of the heat of said metatarsal bone.

2. Prosthetic implant according to claim 1, characterized in that the part shaped like a truncated cylinder (7) of the metatarsal prosthesis (5) is internally striated.

3. Prosthetic implant according to claims 1 or 2, characterized in that the phalangeal prosthesis (1) and the metatarsal prosthesis (5) are made from fluorocarbon resins, carbon fibres or a metal accepted by the organism.

4. Prosthetic implant according to claim 3, characterized in that the metatarsal prosthesis is metallic.

5. Prosthetic implant according to any one of the claims 1 to 4, characterized in that at least one steel wire locking loop is associated with the phalangeal prosthesis.

6. Prosthetic implant according to any one of the claims 1 to 5, characterized in that the metatarsal prosthesis has a fixing spindle with a striated contour arranged centrally and substantially perpendicular to the base of the prosthesis.

7. Drilling device for bone surgery and in particular for fitting the prosthetic implant according to claim 1, comprising a rod (25) provided at one of its ends with a grip (26) and having at its other end a tubular drill (30) constituted by a tube having on one of its edges a series of teeth (31) intended for cutting purposes, characterized in that it also comprises an inner central drill (27) fixed to the rod (25) and constituted by a solid cylinder (28) placed within the tubular drill (30) and connected to the latter and having on its end face a series of radially extending teeth (29), as well as optionally a central bit (32) mounted in the inner central drill (27) and fixed in said rod (25).

8. Device according to claim 7, characterized in that the teeth of the tubular drill (30) and those of the internal central drill (27) are directed from the same side.

9. Device according to either of the claims 7 and 8, characterized in that the teeth of the two drills are sawtooth or have cut faces.

10. Device according to any one of the claims 7 to 9, characterized in that the tubular drill (30) is mounted on the internal central drill (27) by means of a screw (15) engaged in a taphole (16) of the cylinder of the internal central drill, said internal drill (27) being positioned with a varying depth in the tubular drill (30).

## Fig.1a

## Fig.1b

## Fig.1c

## Fig.2a

## Fig.2b

## Fig.2c

## Fig.3

## Fig.4

FIG. 6

FIG. 5

FIG. 7

FIG. 8